# EUROPEAN PATENT APPLICATION

(11) **EP 2 003 115 A1**
(43) Date of publication of application: **17.12.2008**
(21) Application number: 07115707.7
(22) Date of filing: 05.09.2007
(51) Int. Cl.: C07C 211/55, C07C 209/68, C09K 15/18, C10M 133/12, C08F 255/10, C08K 5/18

(54) **Para-alkylated diphenylamines, their synthesis and use as additives**

(30) Priority: 14.06.2007 US 762803
(71) Applicant: Infineum International Limited, Abingdon, Oxfordshire OX13 6BB (GB)
(72) Inventor: Arrowsmith, Stephen, Abingdon, Oxfordshire, OX3 6BB (GB); Booth, Christopher, James, Abingdon, Oxfordshire, OX3 6BB (GB); Burrows, Aubrey, L., Abingdon, Oxfordshire, OX3 6BB (GB); Wangner, Ronald, P., Linden, New Jersey 07036 (US)
(74) Representative: Lewis, Pauline Therese

(57) **Abstract**

Para-alkylated diphenylamine antioxidants arc made by catalytically alkylating diphenylamine with an alkylating agent in the form of a mixture of branched-chain oligomers of butene, the alkylating agent having an average molecular weight in the range of 140 to 300 and a methylvinylidene isomer content of no greater than 10%.

## Description

### FIELD OF THE INVENTION

This invention relates to para-alkylated diphenylamines, useful for example as anti-oxidants in organic liquids, and their preparation.

### BACKGROUND OF THE INVENTION

Alkylated diphenylamines are well-known anti-oxidants such as for use as additive components in lubricating oil compositions (or lubricants) for lubricating the crankcase of spark-ignited or compression-ignited internal combustion engines.

The art describes making them by catalytically alkylating diphenylamine by means of an alkene (or alkylene) alkylating agent. Among the alkylating agents described in the art there are mentioned oligomers of butene, also referred to as butylene.

US-A-5,750,787 describes alkylating diphenylamine with diisobutylene in the presence of a clay catalyst to give a mixture of octyl-substituted diphenylamines.

US-B-6,355,839 ('839) describes alkylating diphenylamine with a mixture of polyisobutylene oligomers containing mostly C₈H₁₆ to C₂₈H₅₆ oligomers (average C₁₄H₂₈) exhibiting a molecular weight in the range of 120 to 160 (average 200), containing a 2-methylvinylidene amount of at least 25% and containing diisobutylene in a fractional amount ranging from 0 to 50%.

US-A-2004/0211113 ('113) describes alkylating diphenylamine with a mixture of polyisobutylene oligomers.

A problem in '839 and '113 is that they both require the use of highly reactive polyisobutylene oligomer fractions.

### SUMMARY OF THE INVENTION

This invention addresses the problem of '839 and '113 by using a less reactive oligomer of butene, thereby providing a simpler and more cost-effective process. Also, the resulting alkylated diphenylamine surprisingly has antioxidancy properties comparable to those of commercially-available alkylated diphenylamines, together with additional properties such as sludge- and varnish-inhibition.

Thus, in a first aspect, the invention comprises a method of preparing a para-alkyl-substituted diphenylamine comprising catalytically alkylating diphenylamine with an alkylating agent in the form of a mixture of branched-chain oligomers of butene, the alkylating agent having an average molecular weight in the range of 140 to 300, such as 170 to 300, and a methylvinylidene isomer content of no greater than 10%. Preferably, the butene contains more n-butene than iso-butene.

In a second aspect, the invention comprises a composition comprising or made by admixing:
(A) an oil of lubricating viscosity; and
(B) as an additive component, a para-alkyl-substituted diphenylamine obtained by or obtainable by the method of the first aspect of the invention.

In a third aspect, the invention comprises a method of reducing oxidation in an internal combustion engine comprising lubricating the crankcase thereof with a composition of the second aspect of the invention in the form of a lubricant where the oil of lubricating viscosity is present in a major amount, and operating the engine.

In a fourth aspect, the invention comprises the use of additive component (B) as defined in the second aspect of the invention, in a crankcase lubricant to provide the lubricant with sludge-inhibiting and/or varnish-inhibiting properties in operation of the lubricant in an internal combustion engine.

In this specification, the following words and expressions, if and when used, have the meanings ascribed below:
"active ingredient" or "(a.i.)" refers to additive material that is not diluent or solvent;
"comprising" or any cognate word specifies the presence of stated features, steps, or integers or components, but does not preclude the presence or addition of one or more other features, steps, integers, components or groups thereof; the expressions "consists of" or "consists essentially of" or cognates may be embraced within "comprises" or cognates, wherein "consists essentially of permits inclusion of substances not materially affecting the characteristics of the composition to which it applies;
"major amount" means in excess of 50 mass % of a composition;
"minor amount" means less than 50 mass % of a composition;
"TBN" means total base number as measured by ASTM D2896.

Furthermore in this specification:
"phosphorus content" is as measured by ASTM D5185;
"sulphated ash content" is as measured by ASTM D874;
"sulphur content" is as measured by ASTM D2622;
"KV 100" means kinematic viscosity at 100°C as measured by ASTM D445.

Also, it will be understood that various components used, essential as well as optimal and customary, may react under conditions of formulation, storage or use and that the invention also provides the product obtainable or obtained as a result of any such reaction.

Further, it is understood that any upper and lower quantity, range and ratio limits set forth herein may be independently combined.

### DETAILED DESCRIPTION OF THE INVENTION

The features of the invention relating, where appropriate, to each and all aspects of the invention, will now be described in more detail as follows:

### PARA-ALKYL-SUBSTITUTED DIPHENYLAMINES

These are diphenylamines alkyl-substituted in at least one of the para positions, i.e. in the 4 position. Typically, they comprise diphenylamines that contain material that is alkyl-substituted in that position and no others, so-called mono-substituted material, and also material that is further alkyl-substituted, namely in the 4 and 4' positions, and in no others, so-called di-substituted material. For example, the ratio of mono-substituted to di-substituted material may be in the range of 70:30 to 30:70 by mass.

Also present may be small quantities of unreacted diphenylamine and trisubstituted material.

As an example of alkylated diphenylamine products, there may be mentioned those where 85% of the substituent alkyl groups, as measured chromeatographically area/area, have more than 8 carbon atoms.

### PREPARATIVE METHOD

The alkylation is carried out catalytically wherein the catalyst used may be a clay catalyst such as known in the art. Preferred are sub-bentonites or bentonites which consist predominantly of the clay mineral montmorillonite. Clays may be used in amounts 1 to 60, preferably 2 to 20, mass% based on the mass of reactant diphenylamine.

Commercially-available clays include the following, identified by their respective trade marks: Filtrol, Retrol, Fulcat, Fulmont and Katalysator. They may include acid-activated or acid-leached clays.

Clays are aluminosilicates: aluminium III cations are bonded to an octahedral arrangement of oxygen anions. Repetition of MO₆ units in two dimensions forms an octahedral layer and, likewise, a tetrahedral layer is formed from SiO₄ units. Clays are classified according to the relative number of tetrahedral and octahedral layers, montmorillonite (mentioned above) having an octahedral layer sandwiched between two tetrahedral layers.

Other catalysts that may be used are Lewis acid catalysts for the Friedel-Crafts alkylation of aromatic compounds. Examples include AlCl₃ and BF₃ and derivatives thereof.

Typically, the alkylation is carried out at a pressure of from 1 to 10 bar and at a temperature of from 120 to 190°C. The ratio (weight:weight) of alkylating agent to diphenylamine may, for example, be in the range of 1.5:1 to 5:1, and the catalyst preferably be present in the range of 1 to 10 per cent by weight, based on the weight of reactant diphenylamine. Suitably, the alkylation may be carried out in an inert atmosphere.

### OIL OF LUBRICATING VISCOSITY (A)

The oil of lubricating viscosity (sometimes referred to as "base stock" or "base oil") is the primary liquid constituent of a lubricant, into which additives and possible other oils are blended, for example to produce a final lubricant (or lubricant composition).

A base oil is useful for making concentrates as well as for making lubricating oil compositions therefrom, and may be selected from natural (vegetable, animal or mineral) and synthetic lubricating oils and mixtures thereof. It may range in viscosity from light distillate mineral oils to heavy lubricating oils such as gas engine oil, mineral lubricating oil, motor vehicle oil and heavy duty diesel oil. Generally the viscosity of the oil ranges from 2 to 30, especially 5 to 20, mm²s⁻¹ at 100°C.

Natural oils include animal and vegetable oils (e.g. castor and lard oil), liquid petroleum oils and hydrorefined, solvent-treated mineral lubricating oils of the paraffinic, naphthenic and mixed paraffinic-naphthenic types. Oils of lubricating viscosity derived from coal or shale are also useful base oils.

Synthetic lubricating oils include hydrocarbon oils such as polymerized and interpolymerized olefins (e.g. polybutylenes, polypropylenes, propylene-isobutylene copolymers, chlorinated polybutylenes, poly(1-hexenes), poly(1-octenes), poly(1-decenes)); alkylbenzenes (e.g. dodecylbenzenes, tetradecylbenzenes, dinonylbenzenes, di(2-ethylhexyl)benzenes); polyphenols (e.g. biphenyls, terphenyls, alkylated polyphenols); and alkylated diphenyl ethers and alkylated diphenyl sulfides and the derivatives, analogues and homologues thereof.

Another suitable class of synthetic lubricating oil comprises esters of dicarboxylic acids (e.g. phthalic acid, succinic acid, alkyl succinic acids and alkenyl succinic acids, maleic acid, azelaic acid, suberic acid, sebasic acid, fumaric acid, adipic acid, linoleic acid dimer, malonic acid, alkylmalonic acids, alkenyl malonic acids) with a variety of alcohols (e.g. butyl alcohol, hexyl alcohol, dodecyl alcohol, 2-ethylhexyl alcohol, ethylene glycol, diethylene glycol monoether, propylene glycol). Specific examples of these esters include dibutyl adipate, di(2-ethylhexyl) sebacate, di-n-hexyl fumarate, dioctyl sebacate, diisooctyl azelate, diisodecyl azelate, dioctyl phthalate, didecyl phthalate, dieicosyl sebacate, the 2-ethylhexyl diester of linoleic acid dimer, and the complex ester formed by reacting one mole of sebacic acid with two moles of tetraethylene glycol and two moles of 2-ethylhexanoic acid.

Esters useful as synthetic oils also include those made from C₅ to C₁₂ monocarboxylic acids and polyols, and polyol ethers such as neopentyl glycol, trimethylolpropane, pentaerythritol, dipentaerythritol and tripentaerythritol.

Unrefined, refined and re-refined oils can be used in the compositions of the present invention. Unrefined oils are those obtained directly from a natural or synthetic source without further purification. For example, a shale oil obtained directly from retorting operations, a petroleum oil obtained directly from distillation or ester oil obtained directly from an esterification process and used without further treatment would be unrefined oil. Refined oils are similar to unrefined oils except that they have been further treated in one or more purification steps to improve one or more properties. Many such purification techniques, such as distillation, solvent extraction, acid or base extraction, filtration and percolation are known to those skilled in the art. Re-refined oils are obtained by processes similar to those used to obtain refined oils from refined oils that have already been used. Such re-refined oils are also known as reclaimed or reprocessed oils and are often additionally processed by techniques for treating spent additive and oil breakdown products.

Other examples of base oil are gas-to-liquid ("GTL") base oils, i.e. the base oil may be an oil derived from Fischer-Tropsch synthesised hydrocarbons made from synthesis gas containing H₂ and CO using a Fischer-Tropsch catalyst. These hydrocarbons typically require further processing to make them useful as base oils. For example, they may, by methods known in the art, need to be hydroisomerized; hydrocracked and hydroisomerized; dewaxed; or hydroisomerized and dewaxed.

Base oil may be categorised in Groups I to V according to the API EOLCS 1509 definition.

The oil of lubricating viscosity may be present in a concentrate-forming amount (e.g., from 30 to 70, such as 40 to 60, mass %) so that the composition is in the form of a concentrate containing for example 1 to 90, such as 10 to 80, preferably 20 to 80, more preferably 20 to 70, mass % active ingredient of an additive or additives, being a para-alkyl-substituted diphenylamine of the invention as sole additives with one or more co-additives.

The oil of lubricating viscosity used in a concentrate is a suitable oleaginous, typically hydrocarbon, carrier fluid, e.g. mineral lubricating oil, or other suitable solvent. Oils of lubricating viscosity such as described herein, as well as aliphatic, naphthenic, and aromatic hydrocarbons, are examples of suitable carrier fluids for concentrates.

Concentrates constitute a convenient means of handling additives before their use, as well as facilitating solution or dispersion of additives in lubricating oil compositions. When preparing a lubricating oil composition that contains more than one type of additive (sometime referred to as "additive components"), each additive may be incorporated separately, each in the form of a concentrate. In many instances, however, it is convenient to provide a so-called additive "package" (also referred to as an "adpack") comprising one or more co-additives, such as described hereinafter, in a single concentrate.

The oil of lubricating viscosity may be provided in a major amount, in combination with a minor amount of at least one additive and, if necessary, one or more co-additives, such as described hereinafter, constituting a lubricating oil composition. This may be accomplished by adding the additive directly to the oil or by adding it in the form of a concentrate thereof to disperse or dissolve the additive. Additives may be added to the oil by methods known to those skilled in the art, either prior to, contemporaneously with, or subsequent to addition of other additives.

The terms "oil-soluble" or "oil-dispersible", or cognate terms, used herein do not necessarily indicate that the compounds or additives are soluble, dissolvable, miscible, or are capable of being suspended in the oil in all proportions. The compounds or additives are, however, soluble or stably dispersible in oil to an extent sufficient for them to exert their intended effect in the environment in which the oil is employed. Moreover, the additional incorporation of other additives may permit incorporation of higher levels of a particular additive, if desired.

The lubricating oil compositions may be used to lubricate mechanical engine components, particularly in internal combustion engines, e.g. spark-ignited or compression-ignited two- or four-stroke reciprocating engines, by adding the composition thereto. Preferably, they are crankcase lubricants.

The lubricating oil compositions and concentrates comprise defined components that may or may not remain the same chemically before and after mixing with an oleaginous carrier. This invention encompasses compositions and concentrates which comprise the defined components before mixing, or after mixing, or both before and after mixing.

When concentrates are used to make the lubricating oil compositions, they may for example be diluted with 3 to 100, e.g. 5 to 40, parts by mass of oil of lubricating viscosity per part by mass of the concentrate.

### CO-ADDITIVES

The composition includes, as indicated above, one or more co-additives to provide certain performance characteristics. As examples there may be mentioned the following, which are known in the art:
Dispersants, including ashless dispersants, the primary function of which is to hold solid and liquid contaminants in suspension.
Detergents in the form of metal salts of acidic organic compounds one of whose functions to reduce piston deposits and which normally have acid-neutralising properties.
Anti-oxidants, or oxidation inhibitors, for example in the form of aromatic amines or hindered phenols, other than para-alkylated-substituted diphenylamines of the invention.
Anti-wear agents such as metal (e.g. Zn) salts of dihydrocarbyl dithiophosphates.
Metal-containing friction modifiers such as molybdenum compounds.
Other co-additives may include one or more of rust and corrosion inhibitors, pour point depressants, anti-foaming agents, emulsifiers and demulsifiers, and viscosity modifiers.

### ENGINE

The invention is applicable to a range of internal combustion engines such as compression-ignited and spark-ignited two- or four-cylinder reciprocating engines. Examples include engines for passenger cars, light commercial vehicles and heavy duty on-highway trucks; engines for aviation, power-generation, locomotive and marine equipment; and heavy duty off-highway engines such as may be used for agriculture, construction and mixing.

### EXAMPLES

This invention will now be particularly described in the following examples which are not intended to limit the scope of the claims hereof.

### PREPARATION OF ANTIOXIDANTS

### Example 1 Oligomeric n-butene-Substituted Diphenylamine

Diphenylamine (400 g; 2.38 mol), aluminium trichloride 40 g (10 % w/w) and an oligomeric n-butene based olefin derived from a low reactivity C₈-C₂₈ stream (1600 g; 4 weight equivalents) were added to a 3.8 litre autoclave. The olefin had a methylvinylidene content of less than 10%. The autoclave was heated to 190°C, pressurised to 10 bar, and the reactants stirred at 500 rpm and heated for 6 hours at this temperature. The reactor was cooled to 80°C and the product removed and treated with sodium hydroxide (10 % w/w) to pH 10. The resulting mixture was centrifuged at 2500 rpm for 25 minutes and the organic layer removed. Excess olefin was removed *in vacuo* to give a black oil which was distilled to give the final product in the form of a para-alkyl-substituted diphenylamine, where the alkyl groups essentially consisted of C₈, C₁₂, C₁₆, C₂₀, C₂₄ and C₂₈ groups. The yield was 1.054 kg.

### FORMULATIONS AND TESTS THEREOF

### Oxidation Testing

The product of Example 1 as a test additive and a commercially-available C₉-substituted diphenylamine as a comparison additive (Example A) were each formulated into petrol car crankcase lubricants in combination with one or more hindered phenol antioxidants, anti-wear additives, ashless dispersants, metal detergents, friction modifiers and Mo-based additives.

Lubricants were formulated from Example 1 and from Example A; each contained 0.7 mass % of the test additive of Example 1 or of the comparison additive, and 200 ppm by mass of Mo. Both lubricants were otherwise identical.

Each of the two lubricants was subjected to ASTM D7097, a "Moderately High Temperature Thermo-Oxidation Engine Oil Simulation" Test or MHT TEOST. The results are set out in the table below:

| **FORMULATION** | LUBRICANT (ADDITIVE) | DEPOSIT (mg) | REPEATABILITY* (mg) |
|---|---|---|---|
| 1 (0.7 mass % test additive; 200 ppm Mo) | 1.A (comparison) | 36 | 7.8 |
| | 1.1 (Ex 1) | 26 | 6.3 |

| | | | |
|---|---|---|---|
| * calculated from the ASTM standard. | | | |

The results are expressed in terms of mg of deposit produced: a lower value indicates a better anti-oxidancy performance, and account is taken of repeatability when comparing results.

The lubricant of the invention 1.1 exhibits anti-oxidancy performance comparable to that of the comparison lubricant (1.A).

The results are surprising, however, because the additive of the invention (Example 1) has a higher molecular weight than the comparison additive (Example A). It therefore has a lower percentage of amine nitrogen and therefore, at the same treat rate, would be expected to give rise to a poorer anti-oxidancy performance.

### Sequence VG Testing [ASTM D6593]

The product of Example 1 as a test additive, and a commercially-available C9-substituted diphenylamine as a comparison additive (Example A) were each formulated into petrol car crankcase lubricants in combination with one or more hindered phenol antioxidants, anti-wear additives, ashless dispersants, metal detergents, friction modifiers, Mo-based additives and viscosity modifiers. The base oil was Group II base oil.

Each lubricant was identical except that one lubricant contained the test additive (Example 1) and the other lubricant contained the comparison additive (Example A). The treat rate of test and comparison additives was 0.70 mass %.

The results are set out in the table below.

| LUBRICANT (ADDITIVE) | AES | RACS | AEV | APSV |
|---|---|---|---|---|
| 2.A (comparison) | 7.05 * | 8.82 | 8.86* | 7.37* |
| 2.1 (Ex 1) | 8.39 | 9.43 | 9.15 | 7.94 |

| | | | | |
|---|---|---|---|---|
| Key to tests: AES: average engine sludge (7.8 minimum to pass) RACS: rocker arm cover sludge (8 minimum to pass) AEV: average engine varnish (8.9 minimum to pass) APSV: average piston skirt varnish (7.5 minimum to pass) * = failing result | | | | |

The results show that, in each of the four tests, the lubricant containing the test additive performed better than the lubricant containing the comparison additive. Also, the former lubricant achieved a "pass" in all four tests whereas the latter "failed" in three of the four tests.

## Claims

1. A method of preparing a para-alkyl-substituted diphenylamine comprising catalytically alkylating diphenylamine with an alkylating agent in the form of a mixture of branched-chain oligomers of butene, the alkylating agent having an average molecular weight in the range of 140 to 300 and a methylvinylidene isomer content of no greater than 10%.

2. A method as claimed in claim 1 wherein the butene contains more n-butene than iso-butene.

3. A method as claimed in claim 1 wherein the diphenylamine is catalytically alkylated using an acid clay or Lewis acid catalyst.

4. A method as claimed in claim 1 wherein the mole ratio of alkylating agent to diphenylamine is in the range of 2:1 to 6:1 and wherein the alkyl groups have an average molecular weight in the range of 170 to 300.

5. A method as claimed in claim 4 wherein the mole ratio of alkylating agent to diphenylamine is in the range of 2:1 to 4:1.

6. A method as claimed in claim 1 wherein the alkylating agent has an average molecular weight in the range of 140 to 300 and a methylvinylidene isomer content of no greater than 10%.

7. A method as claimed in claim 6 wherein the alkylating agent has an average molecular weight in the range of 170 to 300.

8. A composition comprising or made by admixing:
(A) an oil of lubricating viscosity; and
(B) as an additive component, a para-alkyl-substituted diphenylamine obtained by or obtainable by the method of claim 1.

9. A composition as claimed in claim 8 further comprising one or more co-additives, different from (B), selected from ashless dispersants, metal detergents, corrosion inhibitors, metal dihydrocarbyl dithiophosphates, antioxidants, pour point depressants, friction modifiers, antifoam agents and viscosity modifiers.

10. A composition as claimed in claim 8 wherein the oil of lubricating viscosity is present in a concentrate-forming amount.

11. A composition as claimed in claim 5 wherein the oil of lubricating viscosity is present in a major amount.

12. A method of reducing oxidation in an internal combustion engine comprising lubricating the crankcase thereof with a composition as claimed in claim 8 and operating the engine.
